(19) Europäisches Patentamt
European Patent Office
Office européen des brevets

(11) EP 2 687 150 A1

(12) **EUROPEAN PATENT APPLICATION**

(43) Date of publication:
22.01.2014 Bulletin 2014/04

(51) Int Cl.:
*A61B 3/12* (2006.01)     *A61B 3/15* (2006.01)
*A61B 3/10* (2006.01)

(21) Application number: 12177376.6

(22) Date of filing: 20.07.2012

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Canon Kabushiki Kaisha
Tokyo 146-8501 (JP)

(72) Inventor: Kowalski, Bartlomiej
42-200 Czestochowa (PL)

(74) Representative: Bury, Lech Marek
Przedsiebiorstwo Rzeczników Patentowych
PATPOL Sp. z o.o.
Nowoursynowska
02-776 Warszawa (PL)

(54) **Method, apparatus and program for detecting a region in an OCT scan of an object affected by a reflection artifact**

(57) The invention provides a method of detecting a region in an OCT scan of an object that is affected by a reflection artifact. The method comprises the steps of: processing the OCT scan to identify a predetermined feature of the object, detecting (S5-S7; S16) an abnormality (10-2; 48) in the identified feature; and using (S8; S16, S17) the detected abnormality to detect the affected region.

Fig. 1

**Description**

The present invention relates to methods, apparatuses and programs for detecting regions in an optical coherence tomography (OCT) scan of an object affected by reflection artifacts.

Optical coherence tomography is a technique for examination of three-dimensional structures of partially transparent matter. According to this technique, low coherent light is divided into two portions. One portion is used as sample light to be passed into a sample path to illuminate the sample under test. The second portion is used as reference light and led through a reference path to be recombined at a point of recombination with the light back-scattered from the sample. The recombined light contains an interferometric signal carrying information about the internal structure of the sample.

One of the typical ways for retrieving information from the recombined light, known as "Fourier domain Optical Coherence Tomography" (FdOCT), is based on spectral analysis of the recombined light. The spectrum of the recombined light, i.e. the distribution of the light intensities for the various spectral components, is recorded. This can be performed by using either a spectrometer as disclosed in "Spectral Optical Coherence Tomography", SOCT; Szkulmowska et al., Journal of Physics D: Applied Physics, Vo. 38, 2005, 2606 - 2611, or a tuned light source as disclosed in "Swept Source Optical Coherence Tomography", SSOCT; see R. Huber et al., Optics Express, Vol. 13, 2005, 3513 - 3528.

Common OCT devices comprise means for scanning the light beam illuminating the object transversally, i.e. in one or two directions perpendicular to said light beam. A two- or three-dimensional image of the internal sample structure is thus generated. A one-dimensional signal recorded along the light beam is usually called "A-scan".

An A-scan provides information about the structure of a sample, further referred to as structural information, in the depth direction of the sample herein also called the z-axis or in-depth axis.

A series of A-scans across the surface of the sample permits a cross-sectional reconstruction of a plane through the anterior or posterior segment of the tissue. This is known as a B-scan or a tomogram.

The series of A-scans from which such a B-scan is obtained are spaced one from the next in a direction perpendicular to the in-depth axis. For the sake of simplicity the direction of collecting consecutive A-scans will be called the x-axis herein. Thus, a B-scan provides structural information of a sample in an X-Z plane.

The direction perpendicular to the x- and z- axes will be called the y-axis herein. Consecutive B-scans may be collected along the y-axis so as to provide a full data set corresponding to a three dimensional (3D) volume or space.

A general problem associated with imaging, complex-conjugate ambiguity, that involves acquiring samples of the signal spectrum, particularly with FdOCT, is the complex ambiguity artifact. In FdOCT a signal is actually acquired as a spectrum of broadband light source modulated by interference fringes. To get the information about depth-structure of a sample, Fourier transformation is applied, followed by resampling of the original signal from wavelength space to wavenumber space. The acquired signal is in the real domain, and accordingly its Fourier transform (FT) has real and imaginary outputs. Basically, a tomogram (B-scan) of a sample can represent real output or imaginary output of FT. The choice which output is shown depends on certain experimental conditions. Anyhow, regardless of which FT output is provided, it can be affected by the second output. For the sake of clarity, we assume here that the tomogram represents real output of FT.

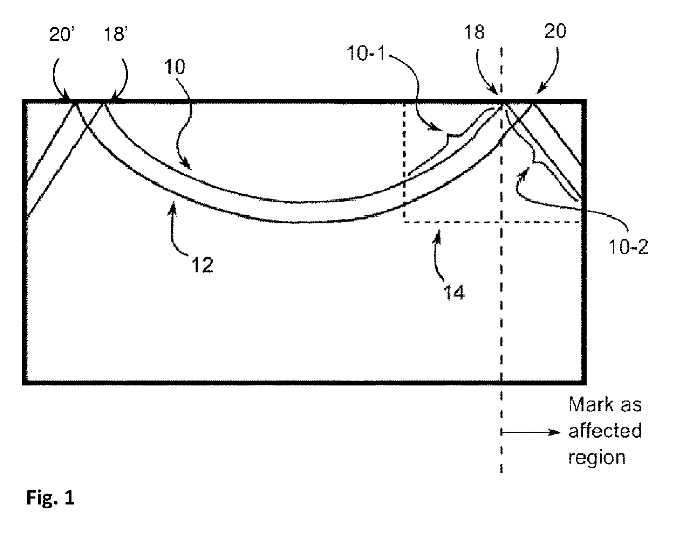

In certain conditions, when the sample has complex morphology (e.g. retina of a human eye), one portion of an object can occur on the imaginary output of the Fourier transform while another portion of the object is present on the real output of Fourier transform. Due to the dual nature of Fourier transform of real signals, the portion of the object present on the imaginary output of the FT will occur on the real output of Fourier transform. Such a signal is observed like a mirror reflection from the edge of the tomogram and sometimes is referred to as a "mirrored image". Therefore the tomogram showing the real output of FT contains some portion of the object that overlaps partly a real image. That effect renders the region where real and mirrored image overlaps ambiguous (Fig. 1).

Typically the result of imaging is used for further examination or diagnosis of the object under test. Typically retina layers are often presented in a form of so called thickness map showing the thickness of all retina layers and typically used for diagnosis of numerous eye pathologies. A portion of tomogram affected by the mirrored image is hard to interpret, analyze and use properly to determine the morphology of the object under test. In case of retina imaging of human eye, the tomograms (structural data) are processed for identification of particular layers of retina providing information about thickness of the retina and/or different layers as well as other clinically useful information.

When structural data contains artifacts, like the one described above, it can provide incorrect information about morphology of the sample and could result in incorrect conclusions by a clinician. Thus, it is desirable to identify structural data with artifacts. It is also desirable to avoid errors of analysis caused by such structural data having artifacts.

A number of approaches have been proposed to solve this problem using sophisticated imaging equipment, for example approaches are disclosed by Bauman et al., in Optics Express, vol.15, no. 20, p.13375 (2007), Targowski et al, in Optics Communications 229, p.79 (2004); or disclosed in EP 2161564 A. They are aimed to obtain full range of imaging free of ambiguity related to real/imaginary outputs of Fourier Transformation. However all these solutions require sophisticated hardware constructions and a number of additional measurements to be taken which significantly increase the time required to obtain an image.

The purpose of invention is to provide a method for detecting unreliable areas in OCT images so as to enhance recognition of the morphology of the object under test. More specifically it is a purpose of present invention to provide a method to find and recognize regions in the image where artifacts are overlapped with respect to actual parts of the object.

Invention provides a method of detecting a region in an OCT scan of an object that is affected by a reflection artifact. The method comprises following steps:

- processing the OCT scan to identify a predetermined feature of the object;

- detecting an abnormality in the identified feature; and

- using the detected abnormality to detect the affected region.

Preferably OCT scan is a B-scan, the predetermined feature is a layer of the object or a boundary of such a layer, and the abnormality is detected when the boundary extends in an abnormal direction.

The abnormal direction preferably is a direction outside a predetermined range of directions. The range may be $0 < \theta < \frac{\pi}{2}$. Alternatively, the range may be $-\frac{\pi}{2} < \theta < 0$.

Detecting the abnormality preferably comprises applying a Hough transform to data representing the OCT scan and using a result of the transform to detect the abnormal direction.

Preferably the method further comprises defining a limited portion of the OCT scan, and confining the identifying of the predetermined feature and/or confining the detection of the abnormality to said limited portion. Advantageously the limited portion is located in or near a corner of the OCT scan on the side corresponding to the side from which light is incident on the object.

Advantageously when the object is an eye, the layer is a predetermined layer of the eye or a boundary of such a layer.

Preferably predetermined layer is the ILM.

Processing the OCT scan to identify the predetermined features advantageously includes resizing of the image.

Also advantageously processing the OCT scan to identify the predetermined features includes application of median filtering.

Also advantageously Processing of the OCT scan to identify the predetermined features includes application of edge detection filtering.

Preferably the method comprises using the detected abnormality to locate a position of the affected region corresponding to the reflection artifact.

Advantageously location of the affected region is the reflection point corresponding to the reflection artifact.

That method preferably comprises identifying a straight line of best fit with a portion of the layer or layer boundary having the abnormal direction and, if the line crosses an edge of the OCT scan from which light is incident on the object at a crossing point within the borders of the OCT scan, determining that the crossing point is such a reflection point.

The scan also advantageously is a C-scan, while the predetermined feature is an edge of a predetermined part of the object, and the abnormality is detected when at least two edges or edge portions corresponding to the object edge are present in the C-scan.

Preferably the object is an eye and the object edge is an edge between the retina and the vitreous humor.

The processing preferably includes at least one median filtering.

Also preferably processing to identify the object edge comprises using a plurality of edge detection filters oriented in different directions.

Advantageously C-scan processed in said processing step is a combined C-scan obtained by combining a plurality of original C-scans.

Preferably a portion of the C-scan outside an innermost one of the edges present is designated as the affected region.

The present invention also provides an apparatus for detecting a region in an OCT scan of an object that is affected by a reflection artifact. The apparatus comprises:

- means for processing the OCT scan to identify a predetermined feature of the object;

- means for detecting an abnormality in the identified feature; and

- means for using the detected abnormality to detect the affected region.

Invention also provides a program which, when executed by a computer or processor, causes the computer or processor to carry out the above method.

Reference will now be made to the accompanying drawings, in which:

Fig. 1 presents a tomogram (B-Scan) of the eye including a reflected area;

Fig. 2 presents a flow chart for use in explaining a reflection artifact detecting method according to a first embodiment of the present invention;

Fig. 3A presents a region of an OCT image containing a reflection artifact;

Fig. 3B presents a representation of the image region of Fig. 3A in Hough space with marked points corresponding to real lines and lines associated with reflection artifacts in the FdOCT image

Fig. 4 is a schematic view for use in explaining how a perpendicular image (C-Scan) representation can be obtained from a series of tomograms (B-scans);

Fig. 5 is a schematic view for use in explaining perpendicular image (C-Scan) averaging in a second embodiment of the present invention;

Fig. 6 illustrates correlation of single perpendicular image (C-Scan) with FdOCT image (B-Scan) series in the second embodiment;

Fig. 7 presents examples of edge detection kernel masks;

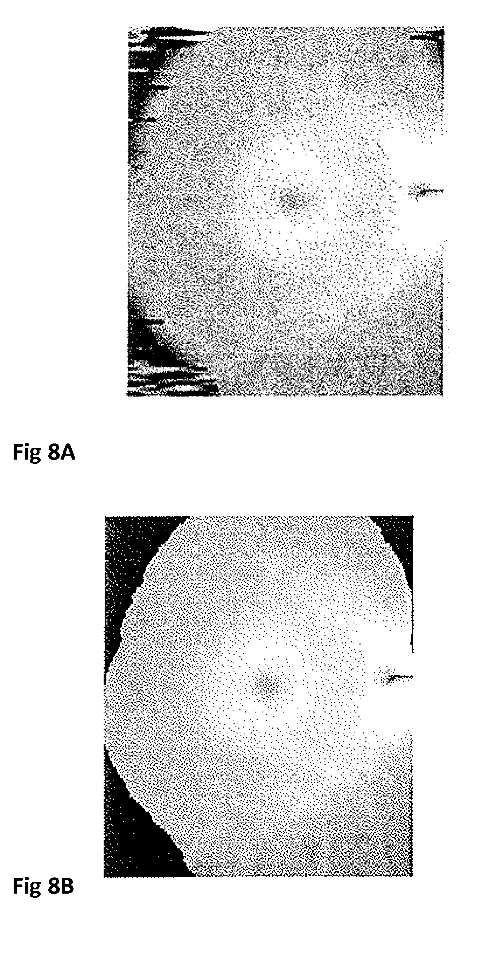

Fig. 8A presents a thickness map generated when detection of reflection artifact affected areas is not applied;

Fig. 8B presents a thickness map generated when detection of reflection artifact affected areas is applied in an embodiment of the present invention; and

Fig. 9 presents a flow chart for use in explaining a reflection artifact detecting method according to the second embodiment of the present invention.

Embodiments of the present invention will now be explained using as an example examination of a human eye. The invention is though not limited to examination of the human eye and is applicable to examination of other kinds of tissue, including a finger or tooth.

In the first embodiment of the present invention, the problem of the reflected image of the retina is solved by analysis of the retina shape on a single B-scan. Referring now to Figure 1, processing techniques can be applied to a B-scan to detect borders (or boundaries) between layers of the retina. Every border corresponds to a boundary between two successive retina layers. Two such borders 10 and 12 are shown in Figure 1. The analysis in the first embodiment makes use of the fact that, in the vicinity of the upper edge of the B-scan, the borders between retina layers resemble the shape of a straight line at least within a limited portion 14 of the tomogram presented in Figure 1. This fact makes it possible to detect the spot 18, 20, where the reflection occurs. There are two reflection points 18 and 20 inside the limited portion 14. It is preferable to find the inner one - 18 - for determination of unreliable areas, and therefore for the sake of simplicity further in this document the inner inner reflection point is meant whenever a reflection point is mentioned.

The process of reflection artifact detection is divided into three phases.

The first phase is an image conditioning which ensures that the orientation of image is the same, regardless of mode of imaging. To explain, in OCT there are possible two modes of operations: vitreous mode and choroid mode. Depending on the mode, the obtained image has normal orientation in which Internal Limiting Membrane (ILM) layer is visible from the top of the image or reverse orientation in which ILM is upside down. The image conditioning simply corrects the images to be always in the normal orientation.

The second phase is further image conditioning aimed to emphasize boundaries of transition from dark to bright area of the signal. It involves processing the tomogram using a series of filtering operations. As a result an image consisting emphasized boundaries between dark and bright areas is obtained. As this embodiment is aimed mainly towards retinal imaging, this phase emphasizes mainly a boundary between the vitreous area which is dark and the retina tissue which is bright. This boundary corresponds to ILM layer and is presented schematically as feature 10 in Fig. 1. In most cases, this second phase emphasizes also a boundary between the Outer Nuclear Layer (ONL) - a dark area of retina tissue - and the IS/OS boundary (junction layer of inner and outer photoreceptor segments), which usually is the brightest layer of the internal retinal structure. This emphasized boundary is denoted as feature 12 in Fig.1.

In the third processing phase, at least a portion of the image resulting from the second phase is subjected to a linear Hough transformation. From the Hough space coefficients (a, b) of a linear equation $z = ax + b$ can be derived. When applied to the limited portion 14 of the tomogram in Figure 1, the Hough transform will return first factors ($a_1$, $b_1$) corresponding to portions 10-1 of the border 10 having a positive gradient ($a_1$ is positive) and second factors ($a_2$, $b_2$) corresponding to portions 10-2 of the border 10 having a negative gradient ($a_2$ is negative). When the coefficients $a$ and $b$ are known, the direction of a linear function is analyzed. The point 18 presented in Fig. 1, where the detected border 10 crosses the upper edge of an image, is marked as a place, where the ILM layer begins to be affected by reflection artifacts. The region to the right of this point 18 is thus identified as a region affected by reflection artifacts. The region may be marked, masked or even subjected to processing to remove the reflection artifacts whilst retaining other actual features of the image. Similarly, other points of reflection including point 20 corresponding to other detected borders including 12 could be also marked, masked or processed in a similar way.

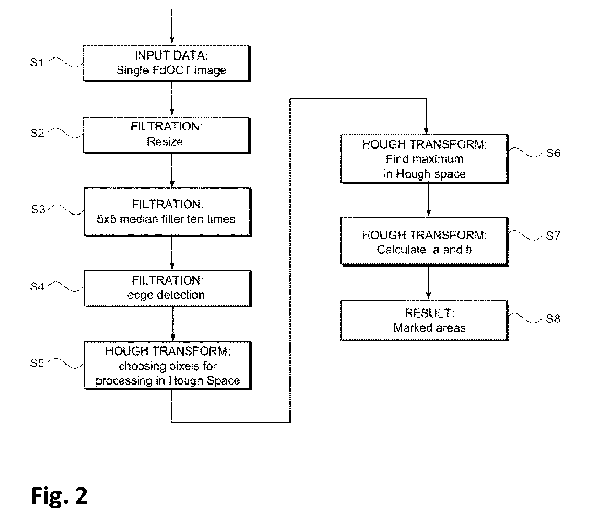

A flow chart of the method according to the first embodiment of the invention is presented in Fig. 2. Input data for analysis is the original B-Scan. At this stage the input data may relate to the entire original B-Scan but preferably it relates to a limited portion of the original B-scan such as the limited portion 14 in Figure 1.

A left limited portion containing the reflection points 18' and 20' can be the left half of the input image, and a right limited portion (corresponding to 14 in Figure 1) containing the reflection points 18 and 20 can be the right half, but the limits of the portions are not strict and can be chosen empirically for best results. The limited portion of the input image can be also reduced in the z-direction to speed-up the processing by reducing the amount of data and to increase robustness. The limit of the or each limited portion in the z-direction may also be chosen empirically for best results.

In one implementation the limited portion 14 located in the upper right corner extends horizontally from a position Xmax/4 in the x-direction to the right side of the tomogram, where Xmax is the width of the image. The left limited portion containing the reflection points 18' and 20' extends horizontally from the left side of the tomogram to the 3Xmax/4. Upper side of both limited portions is aligned to the top of the tomogram, while total height of both portions is approximately 15% of the tomogram height.

Thanks to such selection of the limited portion only a narrow bar aligned to the one of top corners of the original image is subjected to the analysis. It reduces the computing time and simultaneously provides that edges located at the opposite corner and having directions similar to any reflection artifacts present in the limited portion are excluded from analysis.

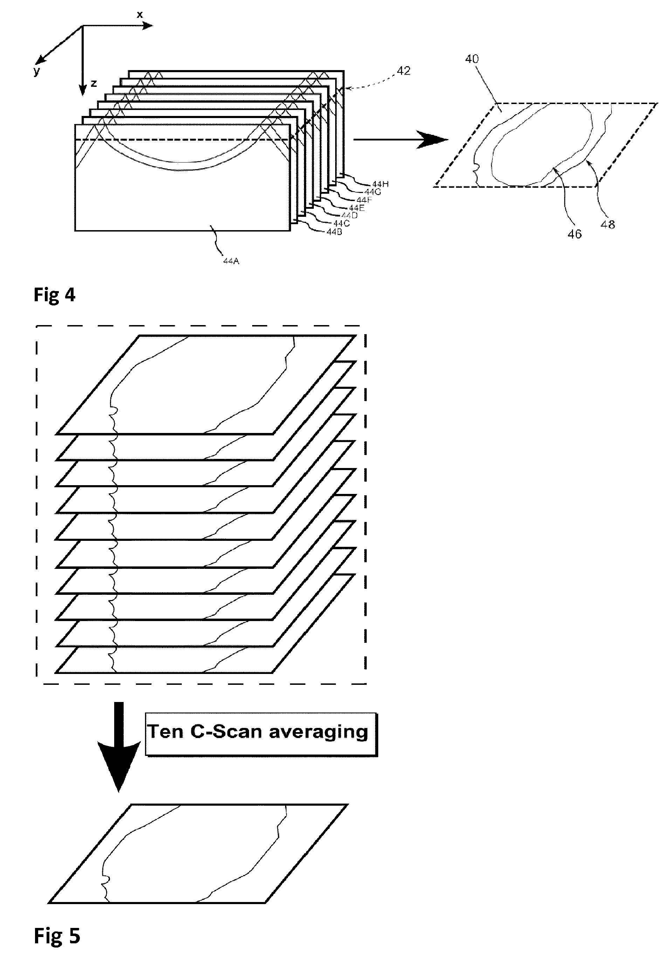

This data is received in step S1. The coordinate system with axes annotation used hereafter is presented in Fig. 4.

The first phase of image conditioning described above is performed before step S1 and will not be described herein in details. The input single FdOCT image or limited portion thereof is prepared in normal orientation prior to step S1.

The second phase of processing is covered by steps S2, S3, S4 and S5. In the step S2 the original image is resized down by a factor of 2 along the $z$ axis, using polyphase resampling. The purpose of this resize is an initial noise reduction and improvement in performance of further calculations. The next step S3 involves further reduction of the image noise by applying ten times a median filter with mask of size 5x5 pixels.

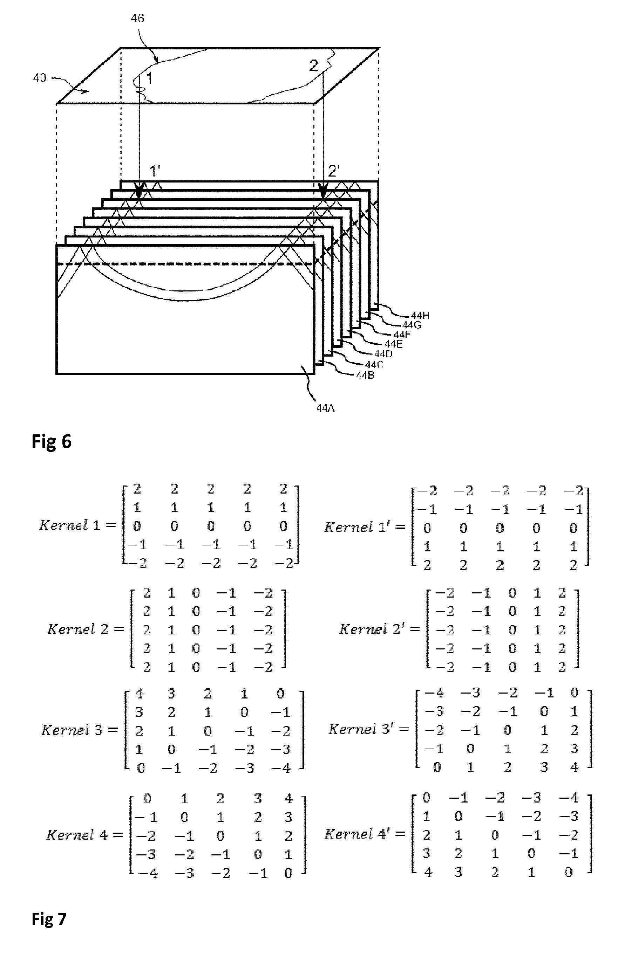

In step S4 the image is subjected to edge detection processing that results in highlighting the edges separating dark and bright areas. Edge detection is run from the top to the bottom of the image and only dark-to-bright edges are detected. The kernel mask used in step S4 is presented in Fig. 6 as kernel 1. The rows of the kernel correspond to the x-direction and the columns of the kernel correspond to the z-direction. The purpose of applying such a large mask is to enhance barely detectable edges, which is useful in case of OCT images of lower quality. Unfortunately, a large filter mask results in thick and imprecise edges. Therefore step S4 also includes processing to reduce the thickness of the detected edges to a single pixel. This processing may be realized using any method from the state of the art, for example superresampling.

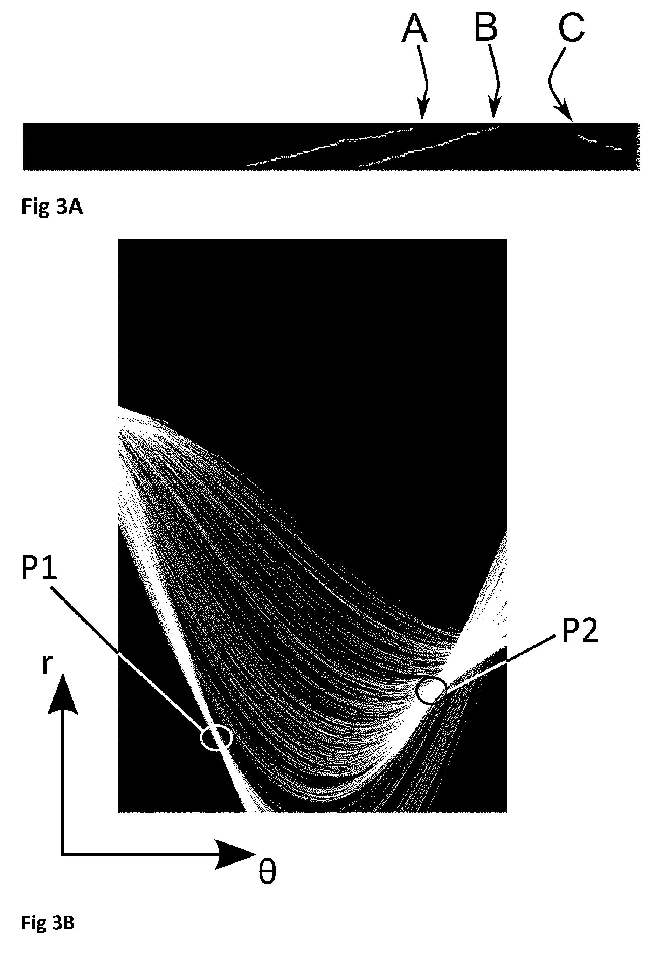

Figure 3A shows an example of the image resulting from the processing in steps S2 to S4. It can be seen that this has edge pixels forming lines A, B and C. Line A corresponds to the line 10-1 presented in Fig. 1, line B corresponds to line 12 in Fig. 1 while line C corresponds to an artifact arising from the Fourier transform processing i.e. line 10-2 in Fig. 1.

The result from step S4 is then input to step S5. Here, if the input data in step S1 was the entire B-Scan then a limited portion (left or right portion of the input image) is now selected for analysis for detecting the reflection point. The left portion is analyzed to detect the reflection point on the left side of the tomogram, while the right portion is analyzed to detect reflection point on the right side of the tomogram. Of course, if the input data in step S1 was already confined to a limited portion then no further selection is needed in S5.

In step S5 the processed data of the limited portion is thereafter transformed to Hough Space. The details of Hough transformation can be found in Shapiro, Linda and Stockman, George: "Computer Vision", Prentice-Hall, Inc. 2001. An example of its application to the limited portion 14 of the B-scan is presented in Fig. 3B. For an arbitrary edge pixel (a pixel forming part of a detected edge) in the image plane of Figure 3A with coordinates ($x_0$,$z_0$), the lines that go through it are the pairs ($r$,$\theta$) with:

$$r\left(\theta\right) = x_0 \cdot cos\theta + z_0 \cdot sin\theta$$

where $r$ (the distance between the line and the origin) is determined by $\theta$.

This corresponds to a sinusoidal curve in the $(r,\theta)$ plane, which is unique to that edge pixel. If the curves corresponding to two edge pixels are superimposed, the location (in the Hough space) where they cross corresponds to a line (in the original image space) that passes through both points. More generally, a set of edge pixels that form a straight line will produce sinusoids which, in the Hough space, cross at the parameters for that line.

In Figure 3B the horizontal axis represents the angle $\theta$ of the line, with angles in the range $0 < \theta < \frac{\pi}{2}$ to

the right hand side and with angles in the range $-\frac{\pi}{2} < \theta < 0$ to

the left hand side. The vertical axis represents the distance r between the angle of the line and the origin.

Then, in step S6, according to the principle of a Hough transform, a value in Hough space corresponding to the substantially largest collinear group of edge pixels is found. As shown in Figure 3B, the largest collinear group of edge pixels in the left hand side is at position P1 and the largest collinear group of edge pixels in the right hand side is at position P2. Such largest collinear group can be identified in a number of ways and selection of the maximal values in the Hough space is just one way. Other ways are described in Shapiro, Linda and Stockman, George. "Computer Vision", Prentice-Hall, Inc. 2001.

The group at P2 corresponds to the edge pixels on line A corresponding to the upper ILM border.

The group at P1 corresponds to edge pixels on line C corresponding to the reflection artifact in Figure 3A. In step S7 the coordinates of this position P1 in Hough space are used to determine the coefficients $a$ and $b$ of line $z = ax + b$ along which said pixels are located (i.e. line C). It is done by means of equations:

$$a = -\frac{\cos(\theta)}{\sin(\theta)}$$

$$b = r/\sin\left(\theta\right)$$

where $r$ and $\theta$ are coordinates of the point in Hough space.

In step S8, the determined line is extended to the upper edge of the image and if it crosses the upper edge at a crossing point between the opposite ends of the upper edge then the crossing point is deemed to be reflection point. If the crossing point is beyond either end of the upper edge then no reflection crossing point is deemed to be present (i.e. there is no region affected by reflection artifacts). This point is marked as a point of reflection. In this embodiment it is only one point that is determined by the identified maximum value of Hough transform result.

When steps S1 - S8 are completed for a first limited portion (14) located near the right upper corner of Fig. 1 and a second limited portion located near the left upper corner of Fig. 1 then both reflection points 18 and 18' are detected and all regions affected with reflection artifacts are marked, i.e. a first region to the right of the reflection point 18 and a second region to the left of the reflection point 18'.

Those skilled in the art will easily recognize that it is possible to use a layer in the eye other than the ILM layer to put the invention into practice. Also numerous alternative techniques known in the art (Shapiro, Linda and Stockman, George. "Computer Vision", Prentice-Hall, Inc. 2001) including edge detection filters, noise reduction techniques and other image processing techniques, to determine direction of the layers in object under test can be applied. The Hough transform itself can be replaced with any other transformation helpful in direction analysis. It is also possible to avoid application of any transformation and just apply linear regression to the detected edge. All such modifications are within the scope of present invention.

As described above, the first embodiment provides a method of detecting a region in an OCT scan of an object that is affected by a reflection artifact, in which the OCT scan is processed to identify a predetermined feature of the object; an abnormality is detected in the identified feature; and the detected abnormality is used to detect the affected region. In this embodiment the OCT scan is a B-scan, the predetermined feature is a layer 10 of the object or a boundary 12 of such a layer, and the abnormality is detected when the layer or layer boundary extends in an abnormal direction.

The abnormal direction is a direction outside the range of directions 0 to $\frac{\pi}{2}$ (this is detected using the Hough transformation); of course, other predetermined ranges can be used instead.

Next, a second embodiment of the invention will be described. In this embodiment so-called C-Scans are used. Referring to Figure 4, a C-scan 40 is an image corresponding to a horizontal cross-section 42 and is generated using structural data taken from a series of B-Scans 44A to 44H. Each C-Scan is taken on a specific height along Y axis. The process of generating a C-Scan can be represented mathematically as:

$$CScan\,(x,y) \; = \; BScan[y](x,z_i)$$

Where $z_i$ corresponds to a point along in-depth axis at which CScan is taken.

In an image perpendicular to the B-scans it is possible to detect the edge 46 between the vitreous humor and the retina on a C-scan image. However, in practice, when reflection artifacts arising from the Fourier transformation are present in the B-scans from which the C-scan is derived, a second edge 48 may be detected. The second embodiment uses this phenomenon (presence of a second edge) in a different approach to detect the reflection points on B-scans by processing a C-scan for detecting the edges 46 and 48.

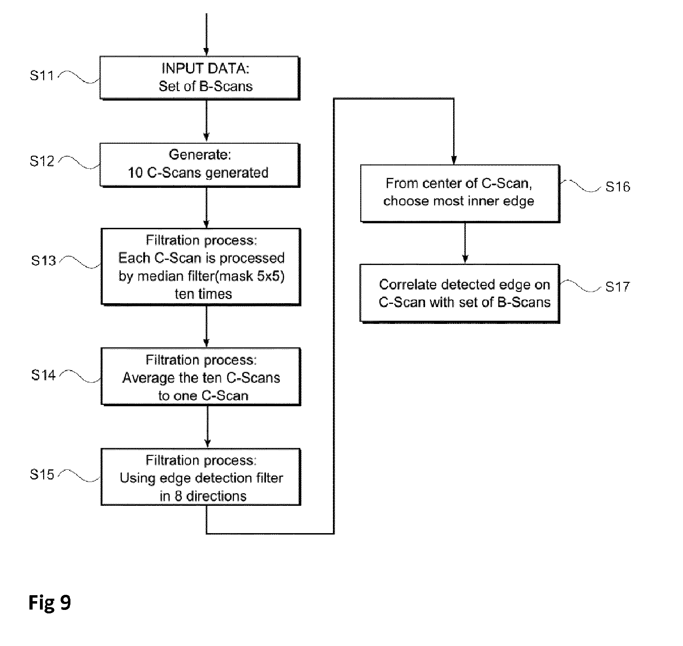

The main steps in the method of the second embodiment will now be described with reference to Figure 9. To increase image quality and to reduce the image noise, before finding the edges 46 and 48 in the C-scan, the C-scan is subjected to a series of filtrations. In a first step S11 ten C-scans are generated, corresponding respectively to ten neighboring z-values. Then in step S12 each C-scan is processed by a median filter with a 5x5 mask ten times. This results in a set of C-scans with reduced noise and also in which individual layers of the eye become indistinct and merge to form a single bright region. After this step, the set of filtered C-scans is averaged to form one C-scan in step S13. This step is shown in Fig. 5.

$$resultingCscan(x,y) \; = \frac{1}{10}\sum_{i=1}^{10} Cscan(x,y))$$

The effect of steps S11 to S13 is to generate a single C-scan with significantly reduced image noise and with areas corresponding to the retina is bright and areas corresponding to the vitreous is dark. In step S14 the resulting C-Scan is then processed with a set of edge detection filters oriented in a number (8 in this example) of predetermined directions. Only edges on image changes from dark to bright are detected, so that if no reflection artifacts are present the bright region is represented by a single edge in the resulting image.

The edge detection masks are shown in Fig. 7. Kernel 1 corresponds to a first vertical direction (from top to bottom). Kernel 1' corresponds to the opposite vertical direction (from bottom to top). Kernel 2 corresponds to a first horizontal direction (from left to right). Kernel 2' corresponds to the opposite horizontal direction (from  right to left). Kernel 3 corresponds to a first diagonal direction (from top left to bottom right). Kernel 3'corresponds to the opposite diagonal direction (from bottom right to top left). Kernel 4 corresponds to a second diagonal direction (from top right to bottom left). Kernel 4' corresponds to the opposite diagonal direction (from bottom left to top right). The output image from this step S14 is an image containing the sharpest edge 46 between the vitreous and retina. This edge 46 is made up of edge portions which must extend in one of the 8 directions.

Presence of the additional edge 48 illustrated in Fig. 4 indicates that reflection artifacts are present. These reflection artifacts should not be allowed to affect the first, inner ILM edge 46. The final step S15 of this method is to map the edge points classified as reflection points from the C-scan to proper tomograms (B-Scan) illustrated in Fig. 6. As B-scan and C-scan are slices of the same cubic 3D data set presented in Fig. 6 the mapping involves keeping track of the height at which C-scan was taken and selecting same reflecting points in the respective B-scans. For example, if C-scan was taken at height $z_i$ and point $(x_j,y_j)$ was identified as located in region affected by reflection then in the B-scan that contains $y_j$ coordinate point $(x_j,z_i)$ belongs to the region affected by reflection.

As described above, the second embodiment provides a method of detecting a region in an OCT scan of an object that is affected by a reflection artifact, in which the OCT scan is processed to identify a predetermined feature of the object; an abnormality is detected in the identified feature; and the detected abnormality is used to detect the affected region. In this embodiment the scan is a C-scan, the predetermined feature is an edge 46/48 of a predetermined part of the object, and the abnormality is detected when at least two edges or edge portions corresponding to the object edge are present in the C-scan. The object is an eye and the object edge 46/48 is an edge between the retina and the vitreous humor.

Although the embodiments presented above refer to FdOCT imaging of an eye persons skilled in the art will easily recognized that invention is also applicable to other objects of well defined shape and layered structure of example a finger is good example of such object.

It will be appreciated that methods embodying the present invention may be computer-implemented methods. Thus, the present invention extends to a program which, when executed by a computer or processor, causes the computer or processor to carry out any of the methods described hereinbefore.

Such a program may be provided by itself or may be carried in, by or on a carrier medium.

The carrier medium may be a storage medium or recording medium, in particular a computer-readable storage medium. Examples include a hard disk drive, DVD, or memory device.

The carrier medium may also be a transmission medium such as a signal. Thus, a program embodying the present invention may be distributed, downloaded or uploaded in the form of a signal through a network including the Internet.

The program may be non-transitory.

**Claims**

1. A method of detecting a region in an OCT scan of an object that is affected by a reflection artifact, the method comprising:

   - processing (S2-S4; S12-S15) the OCT scan to identify a predetermined feature (10, 12; 46, 48) of the object;
   - detecting (S5-S7; S16) an abnormality (10-2; 48) in the identified feature; and
   - using (S8; S16, S17) the detected abnormality to detect the affected region.

2. A method according to claim 1, wherein the OCT scan is a B-scan, the predetermined feature is a layer (10) of the object or a boundary of such a layer, and the abnormality is detected when the layer or boundary extends in an abnormal direction.

3. A method according to claim 2, wherein the abnormal direction is a direction outside a predetermined range of directions $\left(0 < \theta < \frac{\pi}{2}\right)$.

4. A method according to claim 2 or 3, wherein detecting the abnormality comprises applying a Hough transform (S5-S7) to data representing the OCT scan and using a result of the transform to detect the abnormal direction (P2).

5. A method according to any preceding claim, further comprising defining a limited portion (14) of the OCT scan, and confining the identifying of the predetermined feature and/or confining the detection of the abnormality to said limited portion.

6. A method according to claim 5, wherein the limited portion (14) is located in or near a corner of the OCT scan on the side corresponding to the side from which light is incident on the object.

7. A method according to claim 2, 3 or 4, wherein the object is an eye and the layer (10, 12) is a predetermined layer (10) of the eye or a boundary (12) of such a layer.

8. A method according to claim 7, wherein the predetermined layer (10) is the ILM.

9. A method according to any preceding claim, comprising using the detected abnormality to locate a position of the affected region corresponding to the reflection artifact.

10. A method according to the claim 9, wherein location of the affected region is the reflection point (18, 18') corresponding to the reflection artifact.

11. A method according to claim 10, comprising identifying a straight line of best fit with a portion of the layer (10) or layer boundary having the abnormal direction and, if the line crosses an edge of the OCT scan from which light is incident on the object at a crossing point within the borders of the OCT scan, determining that the crossing point is such a reflection point (18, 18').

12. A method according to claim 1, wherein the scan is a C-scan, the predetermined feature is an edge (46, 48) of a predetermined part of the object, and the abnormality is detected when at least two edges or edge portions corresponding to the object edge are present in the C-scan.

13. A method according to claim 12, wherein the object is an eye and the object edge (46, 48) is an edge between the retina and the vitreous humor.

14. A method according to any one of claim 12 or 13, wherein a portion of the C-scan outside an innermost one of the edges present is designated as the affected region.

15. Apparatus for detecting a region in an OCT scan of an object that is affected by a reflection artifact, the apparatus comprising:

- means for processing (S2-S4; S12-S15) the OCT scan to identify a predetermined feature (10, 12; 46, 48) of the object;
- means for detecting (S5-S7; S16) an abnormality (10-2; 48) in the identified feature; and
- means for using (S8; S16, S17) the detected abnormality to detect the affected region.

16. A program which, when executed by a computer or processor, causes the computer or processor to carry out the method of any one of claims 1 to 15.

**Fig. 1**

S1 — INPUT DATA:
Single FdOCT image

S2 — FILTRATION:
Resize

S3 — FILTRATION:
5x5 median filter ten times

S4 — FILTRATION:
edge detection

S5 — HOUGH TRANSFORM:
choosing pixels for
processing in Hough Space

HOUGH TRANSFORM:
Find maximum
in Hough space — S6

HOUGH TRANSFORM:
Calculate a and b — S7

RESULT:
Marked areas — S8

**Fig. 2**

**Fig 3A**

**Fig 3B**

**Fig 4**

Ten C-Scan averaging

**Fig 5**

**Fig 6**

$$
Kernel\ 1 = \begin{bmatrix} 2 & 2 & 2 & 2 & 2 \\ 1 & 1 & 1 & 1 & 1 \\ 0 & 0 & 0 & 0 & 0 \\ -1 & -1 & -1 & -1 & -1 \\ -2 & -2 & -2 & -2 & -2 \end{bmatrix}
\qquad
Kernel\ 1' = \begin{bmatrix} -2 & -2 & -2 & -2 & -2 \\ -1 & -1 & -1 & -1 & -1 \\ 0 & 0 & 0 & 0 & 0 \\ 1 & 1 & 1 & 1 & 1 \\ 2 & 2 & 2 & 2 & 2 \end{bmatrix}
$$

$$
Kernel\ 2 = \begin{bmatrix} 2 & 1 & 0 & -1 & -2 \\ 2 & 1 & 0 & -1 & -2 \\ 2 & 1 & 0 & -1 & -2 \\ 2 & 1 & 0 & -1 & -2 \\ 2 & 1 & 0 & -1 & -2 \end{bmatrix}
\qquad
Kernel\ 2' = \begin{bmatrix} -2 & -1 & 0 & 1 & 2 \\ -2 & -1 & 0 & 1 & 2 \\ -2 & -1 & 0 & 1 & 2 \\ -2 & -1 & 0 & 1 & 2 \\ -2 & -1 & 0 & 1 & 2 \end{bmatrix}
$$

$$
Kernel\ 3 = \begin{bmatrix} 4 & 3 & 2 & 1 & 0 \\ 3 & 2 & 1 & 0 & -1 \\ 2 & 1 & 0 & -1 & -2 \\ 1 & 0 & -1 & -2 & -3 \\ 0 & -1 & -2 & -3 & -4 \end{bmatrix}
\qquad
Kernel\ 3' = \begin{bmatrix} -4 & -3 & -2 & -1 & 0 \\ -3 & -2 & -1 & 0 & 1 \\ -2 & -1 & 0 & 1 & 2 \\ -1 & 0 & 1 & 2 & 3 \\ 0 & 1 & 2 & 3 & 4 \end{bmatrix}
$$

$$
Kernel\ 4 = \begin{bmatrix} 0 & 1 & 2 & 3 & 4 \\ -1 & 0 & 1 & 2 & 3 \\ -2 & -1 & 0 & 1 & 2 \\ -3 & -2 & -1 & 0 & 1 \\ -4 & -3 & -2 & -1 & 0 \end{bmatrix}
\qquad
Kernel\ 4' = \begin{bmatrix} 0 & -1 & -2 & -3 & -4 \\ 1 & 0 & -1 & -2 & -3 \\ 2 & 1 & 0 & -1 & -2 \\ 3 & 2 & 1 & 0 & -1 \\ 4 & 3 & 2 & 1 & 0 \end{bmatrix}
$$

**Fig 7**

**Fig 8A**

**Fig 8B**

S11 — INPUT DATA:
Set of B-Scans

S12 — Generate:
10 C-Scans generated

S13 — Filtration process:
Each C-Scan is processed
by median filter(mask 5x5)
ten times

S14 — Filtration process:
Average the ten C-Scans
to one C-Scan

S15 — Filtration process:
Using edge detection filter
in 8 directions

From center of C-Scan,
choose most inner edge — S16

Correlate detected edge on
C-Scan with set of B-Scans — S17

**Fig 9**

**EUROPEAN SEARCH REPORT**

Application Number

EP 12 17 7376

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/071404 A1 (SCHMITT JOSEPH M [US] ET AL) 24 March 2011 (2011-03-24)<br>* abstract *<br>* paragraph [0059] - paragraph [0074] *<br>* figure 6a *<br>----- | 1-5,9, 10,15,16 | INV.<br>A61B3/12<br>A61B3/15<br>A61B3/10 |

TECHNICAL FIELDS SEARCHED (IPC)

A61B

The present search report has been drawn up for all claims

| Place of search | Date of completion of the search | Examiner |
|---|---|---|
| The Hague | 17 January 2013 | Marteau, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 17 7376

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2013

| Patent document cited in search report | Publication date | Patent family member(s) | Publication date |
|---|---|---|---|
| US 2011071404 A1 | 24-03-2011 | AU 2010298333 A1<br>CA 2765407 A1<br>EP 2480124 A2<br>US 2011071404 A1<br>WO 2011038044 A2 | 19-01-2012<br>31-03-2011<br>01-08-2012<br>24-03-2011<br>31-03-2011 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2161564 A

**Non-patent literature cited in the description**

- **SZKULMOWSKA et al.** Spectral Optical Coherence Tomography. *Journal of Physics D: Applied Physics,* 2005, vol. 38, 2606-2611
- **R. HUBER et al.** Swept Source Optical Coherence Tomography. *Optics Express,* 2005, vol. 13, 3513-3528
- **BAUMAN et al.** *Optics Express,* 2007, vol. 15 (20), 13375
- **TARGOWSKI et al.** *Optics Communications,* 2004, vol. 229, 79
- **SHAPIRO ; LINDA ; STOCKMAN ; GEORGE.** Computer Vision. Prentice-Hall, Inc, 2001